# FASCICULE DE BREVET EUROPEEN

(11) **EP 1 562 659 B1**
(45) Date de publication et mention de la délivrance du brevet: **18.06.2008**
(21) Numéro de dépôt: 03786034.3
(22) Date de dépôt: 20.11.2003
(51) Int. Cl.: A61M 5/158, A61M 5/32

(54) **DISPOSITIF ANTI-PIQUE POUR AIGUILLE D INJECTION COUDEE**
ANTI-STECHVORRICHTUNG FÜR EINE ABGEWINKELTE INJEKTIONSNADEL
ANTI-STICK DEVICE FOR BENT INJECTION NEEDLE

(30) Priorité: 21.11.2002 FR 0214578
(43) Date de publication de la demande: 17.08.2005
(73) Titulaire: VYGON, F-95440 Ecouen (FR)
(72) Inventeur: HUET, Jean-Max, F-92110 Clichy (FR)
(74) Mandataire: Texier, Christian
(86) Numéro de dépôt international: PCT/FR2003/003435
(87) Numéro de publication internationale: WO 2004/047889

(56) Documents cités:
- FR-A- 2 803 529
- US-A- 5 951 522

## Description

L'invention concerne un dispositif anti-pique pour manipuler avec sécurité une aiguille d'injection à travers la peau, cette aiguille étant coudée et présentant une branche distale perforante et une branche proximale d'alimentation qui forme un coude avec la branche perforante (aiguille de HUBER).

Des exemples d'un dispositif anti-pique pour ce type d'aiguille sont décrits dans la publication FR 2 803 529 et dans le brevet US 5 951 522.

Le dispositif décrit dans la publication FR 2 803 529 est constitué par une paroi formée de panneaux articulés qui permettent à la paroi d'être mise dans une configuration où l'un des panneaux (ou panneau porte-aiguille) est rabattu sur un autre panneau (ou panneau de base) et où un troisième panneau (ou panneau de recouvrement) est rabattu sur le panneau porte-aiguille et fixé à lui, et d'être mise dans une configuration où le panneau porte-aiguille et le panneau de recouvrement fixés l'un à l'autre sont écartés du panneau de base et ménagent entre eux et lui un espace suffisant pour contenir la branche distale de l'aiguille, le panneau de base et le panneau porte-aiguille présentant des perforations respectives qui permettent le passage de la branche distale de l'aiguille et qui coïncident lorsque les panneaux sont appliqués l'un sur l'autre en sorte que la branche distale puisse être introduite dans les perforations des panneaux rabattus l'un sur l'autre jusqu'à ce que la branche proximale de l'aiguille repose sur le panneau porte-aiguille, le panneau de recouvrement étant apte à recouvrir la branche proximale de l'aiguille lorsqu'il est rabattu sur le panneau porte-aiguille, le panneau de base déterminant une zone centrale comportant ladite perforation du panneau et quatre branches latérales deux à deux opposées et deux à deux perpendiculaires, et le panneau porte-aiguille formant deux oreilles latérales relevables pour servir à la préhension manuelle du dispositif lors de la ponction et lors du retrait de l'aiguille.

La présente invention concerne un mode de réalisation de ce dispositif spécialement adapté pour la perfusion d'une chambre implantée sous la peau. Dans ce cas, l'aiguille doit traverser un septum qui est un disque de silicone situé au sommet du dôme incurvé de la chambre. Le dispositif doit assurer la bonne auto obturation du septum au retrait de l'aiguille. A titre d'exemple, on doit pouvoir réaliser 3500 ponctions dans un septum de 1,3 cm2 et 3500 ponctions dans un septum de 0,63 cm². Compte tenu de ce qui précède, il faut pousser très fort sur l'aiguille lors de la pique et tirer très fort lors du retrait. Cette traction lors du retrait nécessite que l'utilisateur applique deux doigts sur la peau du patient de chaque côté de l'aiguille pour maintenir la chambre implantable. Or compte tenu de l'effort important, il peut y avoir des phénomènes de rebond, ce qui peut conduire l'opérateur à se piquer un doigt.

L'invention a pour objet une réalisation simple et peu coûteuse du dispositif défini plus haut qui permet de pousser fort l'aiguille et de tirer fort l'aiguille sans risque de pique pour l'opérateur.

La réalisation de l'invention est notamment caractérisée en ce que le panneau central est conformé de fabrication en sorte que deux branches latérales opposées de ce panneau aient une courbure facilitant l'application de ces branches sur la peau au droit de la chambre implantée et en sorte que les deux autres branches opposées du panneau soient courbables à la demande sous la pression de deux doigts d'une main pour appuyer ces branches sur la peau afin de maintenir la chambre implantée lorsque l'opérateur retire l'aiguille avec son autre main, et en ce que le panneau porte-aiguille et le panneau de recouvrement sont jointifs respectivement de l'une et de l'autre des branches précourbées du panneau de base et présentent de fabrication une courbure en sens inverse de la courbure desdites branches de façon à épouser la courbure des branches lorsqu'ils sont rabattus sur le panneau de base.

On décrira ci-après un exemple d'une telle réalisation, en référence aux figures du dessin joint sur lequel :
- la figure 1 est une vue du dispositif à plat avant rabattement et mise en place de l'aiguille ;
- la figure 2 est une vue du dispositif après mise en place de l'aiguille et avant que le panneau de recouvrement soit rabattu et collé sur le panneau porte-aiguille ;

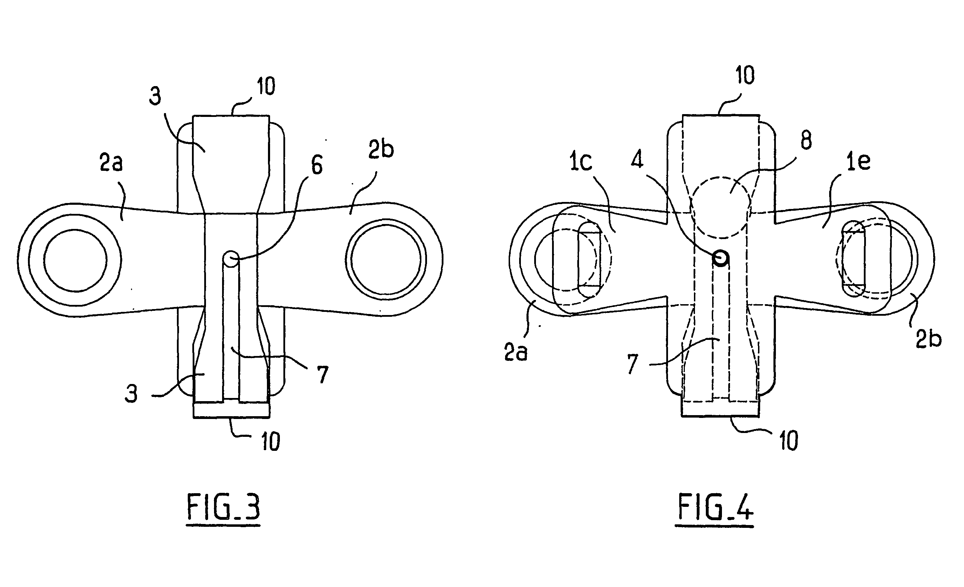

- les figures 3 et 4 sont des vues, respectivement de dessus et de dessous du dispositif après rabattement et collage du panneau de recouvrement ;
- les figures 5 et 6 sont des perspectives latérales du dispositif, respectivement avant et après redressement des ailettes de préhension ;
- la figure 7 est une perspective du dispositif prêt à l'emploi ;
- les figures 8 à 12 montrent des phases successives des opérations de ponction et d'extraction de l'aiguille, et
- les figures 13 et 14 sont des perspectives du dispositif, selon deux angles de vue, après extraction de l'aiguille.

Le dispositif est constitué par une paroi découpée dans une feuille de matière plastique souple et préformée.

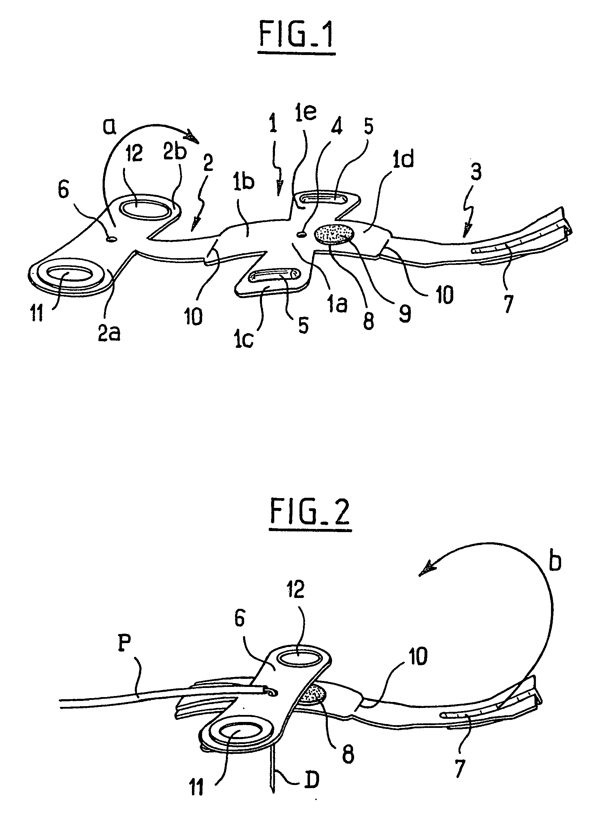

La feuille définit (fig.1) :
- un panneau de base (1) cruciforme qui comprend une zone centrale (la) et quatre bras (1b, 1c, 1d, 1^{e}) deux à deux opposés et deux à deux perpendiculaires qui rayonnent autour de la zone centrale ;
- un panneau porte-aiguille (2) qui prolonge le bras (1b) du panneau de base et lui est articulé par une ligne de pliage (10) ;
- un panneau de recouvrement (3) qui prolonge le bras (1d) du panneau de base et lui est articulé par une ligne de pliage (10) ;

La zone centrale (la) du panneau de base présente une perforation (4) pour le passage de la branche distale biseautée (D) d'une aiguille coudée à angle droit (aiguille de Hubner).

Les bras (1b, 1d) du panneau de base ont une courbure préformée choisie pour que le panneau puisse épouser la forme du dôme d'une chambre implantée.

Les bras (1c, 1e) du panneau de base sont courbables pour pouvoir être appliqués sur la peau au dessus du dôme par une pression manuelle. Avantageusement, ces bras comportent des reliefs (5) pour faciliter l'application des doigts du manipulateur.

Le panneau porte-aiguille (2) et le panneau de recouvrement (3) sont préformés avec une courbure inverse de celle des bras (1b, 1d) pour que ces panneaux épousent ces bras lorsqu'ils sont rabattus sur le panneau central.

Le panneau porte-aiguille (2) présente une perforation (6) pour le passage de la branche distale biseautée (D) de l'aiguille coudée forme, une gouttière (7) pour recevoir 1a branche proximale (P) de l'aiguille et comporte deux oreilles (2a, 2b) relevables pour servir à la préhension du dispositif.

La gouttière obtenue par déformation de la feuille épouse la courbure du panneau de recouvrement. Elle sert à recouvrir 1a zone de liaison entre les deux branches de l'aiguille, la branche proximale (P) de l'aiguille et l'extrémité distale du tube souple (S) qui prolonge l'aiguille.

En outre, elle sert de réservoir de colle.

Le panneau de base (1) est muni à proximité de la perforation (4) d'un disque (8) en matière plastique dure qui est rapporté dans une perforation du panneau et dont la surface a un relief approprié (9), par exemple des stries ou un quadrillage, pour retenir la pointe biseautée de l'aiguille lorsque cette pointe a été amenée au contact de cette surface après retrait de la branche distale de l'aiguille à l'intérieur du dispositif.

Les pliures d'articulation (10) entre panneaux sont constituées par des amincissements locaux de la paroi.

Les oreilles (2a, 2b) du panneau porte-aiguille sont avantageusement munies de moyens, par exemple un relief (11) et un creux (12), aptes à coopérer pour maintenir les deux ailettes appliquées l'une contre l'autre, quand cela est désirable pour éviter un glissement d'une ailette par rapport à l'autre.

Le dispositif est livré à l'utilisateur dans une pochette (stérile) où la feuille est à plat, les panneaux se trouvant sensiblement comme représentés sur la figure 1. La pochette peut contenir également l'aiguille dont la branche distale est protégée provisoirement par un capuchon amovible.

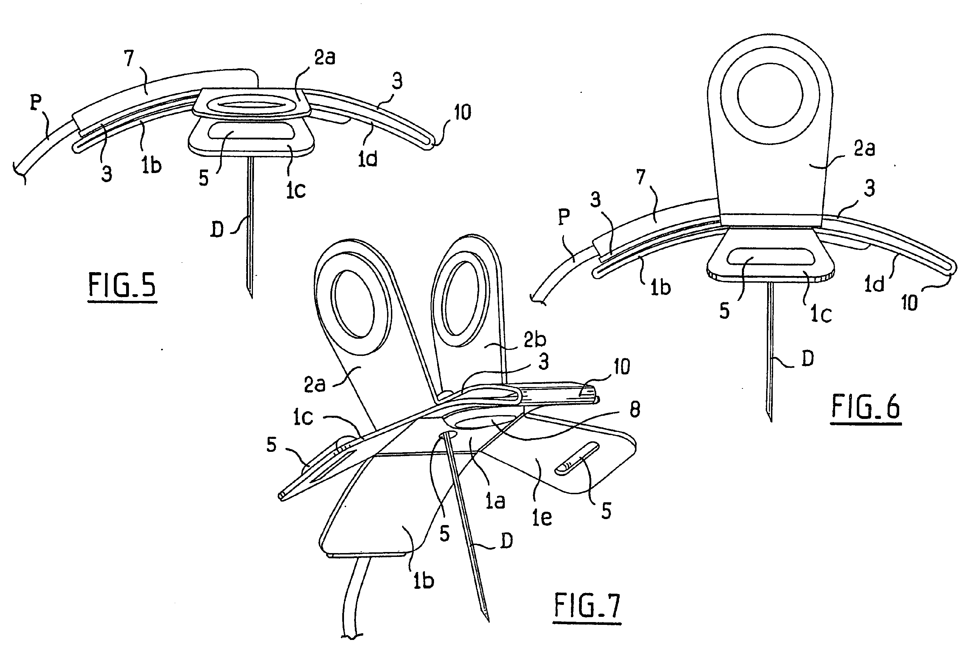

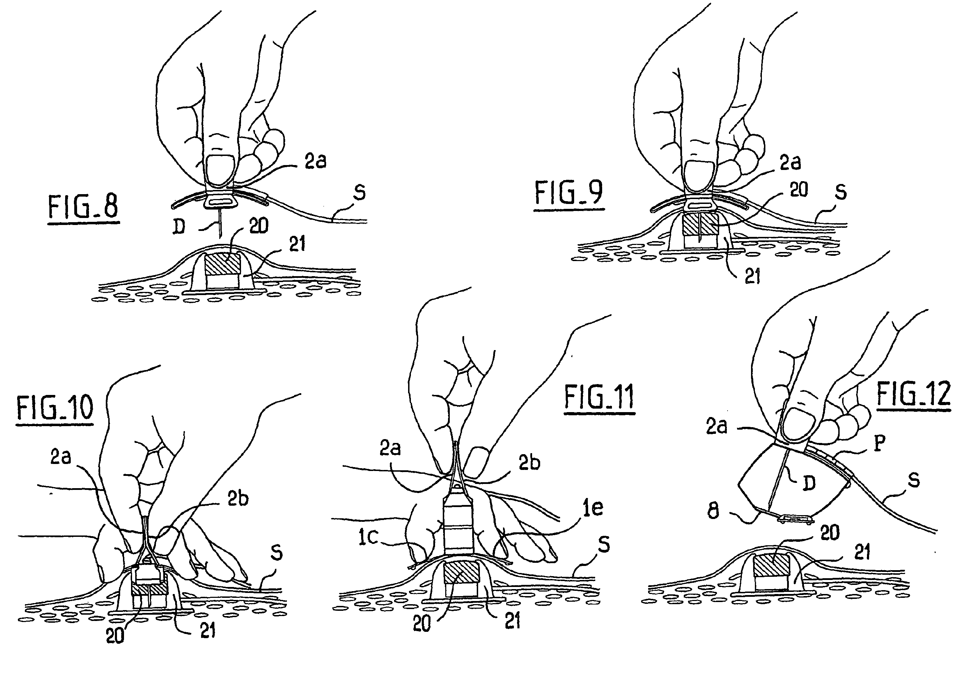

La mise en volume du dispositif en vue de son utilisation est la suivante :
- rabattement du panneau porte-aiguille (2) sur le panneau de base (1) et introduction de la branche distale (D) dans les perforations superposées (4, 6) des deux panneaux (fig.2),
- dépôt de colle dans la gouttière et rabattement du panneau de recouvrement (3) sur le panneau porte-aiguille (2) en sorte que la gouttière (7) recouvre la branche proximale (P) de l'aiguille.
- relèvement des oreilles (2a, 2b) du panneau porte-aiguille (figs. 6 et 7).
- prise du dispositif entre les deux doigts d'une main par les deux oreilles pressées l'une contre l'autre (Fig.8) .
- ponction de la peau au droit du dôme (20) de la chambre implantée (21) avec une force suffisante pour que la pointe de l'aiguille pénètre à force dans la chambre (Fig.9).
- rabattement des oreilles sur la peau et maintien du dispositif au moyen d'un pansement ou similaire pour le remplissage de la chambre.

Pour extraire l'aiguille après remplissage de la chambre, l'opérateur relève les oreilles du dispositif, les saisit et appuie avec son autre main sur les panneaux (1c, 1e) lesquels du fait de leur courbure sont appliqués sur la peau à l'endroit de la chambre implantée pour maintenir la chambre implantée pendant qu'il extrait l'aiguille (Fig. 10 et 11) en tirant sur les ailettes.

La partie centrale (la) du panneau se relève et prend une courbure inverse. Ceci est rendu possible par le profil en croix du panneau et les articulations du panneau avec les deux autres panneaux.

La partie supérieure du dispositif se courbe fortement en partie avant, la partie arrière ne peut se courber, car elle est rigidifiée par le collage et la branche distale de l'aiguille. Tout ceci permet de créer élastiquement, entre la partie inférieure et la partie supérieure du dispositif un espace dans lequel la branche distale de l'aiguille peut s'escamoter.

Au fur et à mesure de la remontée, des deux branches de l'aiguille l'angle d'environ 90° initial, se réduit. Ainsi lors du dégagement complet de la branche distale de l'aiguille celle-ci se détend en partie avant et vient se planter dans le disque.

L'élasticité de la déformation des deux parties (inférieure et supérieure) et le quadrillage en relief du disque empêchent tout retour en arrière, tout déplacement de la branche distale de l'aiguille et bien sûr tout risque de pique ou de réutilisation de l'aiguille.

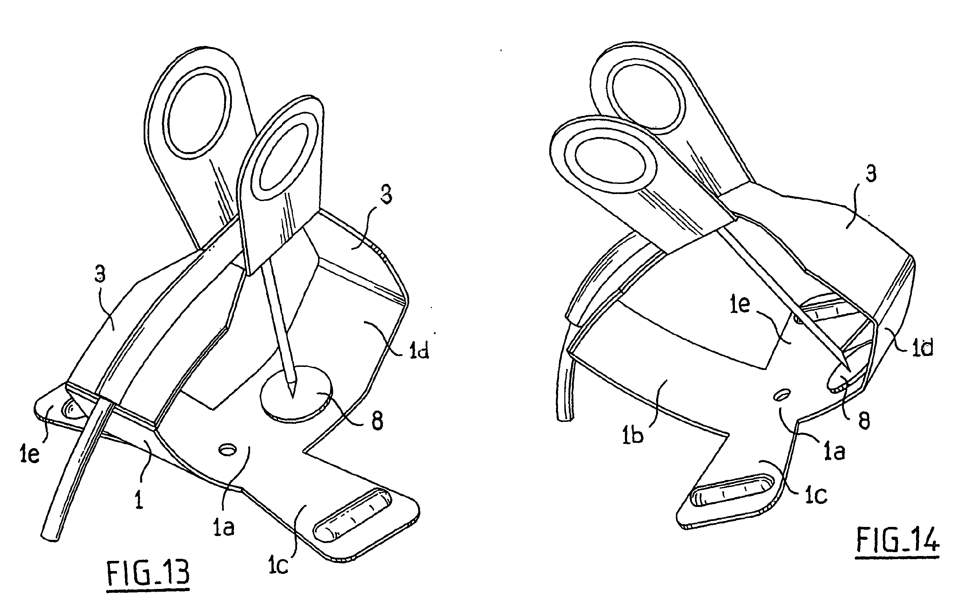

Les figures 12 et 13 montrent l'aiguille en butée contre le disque dur (8) à l'intérieur du dispositif.

L'invention n'est pas limitée à cet exemple d'une réalisation conforme à l'invention.

## Revendications

1. Dispositif anti-pique pour manipuler avec sécurité une aiguille d'injection à travers la peau en vue d'alimenter une chambre implantée sous la peau, cette aiguille étant coudée et présentant une branche distale perforante et une branche proximale d'alimentation qui forme un coude avec la branche perforante, ce dispositif étant constitué par une paroi formée de panneaux articulés (1, 2, 3) qui permettent à la paroi d'être mise dans une configuration où l'un des panneaux dit panneau porte-aiguille (2) est rabattu sur un autre panneau (1) dit panneau de base et où un troisième panneau (3) dit panneau de recouvrement est rabattu sur le panneau porte-aiguille et fixé à lui, et d'être mise dans une configuration où le panneau porte-aiguille et le panneau de recouvrement fixés l'un à l'autre sont écartés du panneau de base et ménagent entre eux et lui un espace suffisant pour contenir la branche distale (D) de l'aiguille, le panneau de base (1) et le panneau porte-aiguille (2)présentant des perforations respectives (4, 6) qui permettent le passage de la branche distale de l'aiguille et qui coïncident lorsque les panneaux sont appliqués l'un sur l'autre en sorte que la branche distale puisse être introduite dans les perforations des panneaux rabattus l'un sur l'autre jusqu'à ce que la branche proximale de l'aiguille repose sur le panneau porte-aiguille, le panneau de recouvrement étant apte à recouvrir la branche proximale (P) de l'aiguille lorsqu'il est rabattu sur le panneau porte-aiguille, le panneau de base (1) déterminant une zone centrale (la) comportant ladite perforation (4) du panneau et quatre branches latérales deux à deux opposées et deux à deux perpendiculaires dont deux branches latérales opposées (1c, 1e) du panneau soient courbables à la demande sous la pression de deux doigts d'une main pour appuyer ces branches sur la peau et la chambre, et le panneau porte-aiguille (2) et le panneau de recouvrement (3) sont jointifs respectivement de l'une et de l'autre des branches (1b, 1d) du panneau de base et le panneau porte-aiguille (2) formant deux oreilles latérales (2a, 2b) relevables pour servir à la préhension manuelle du dispositif lors de la ponction et lors du retrait de l'aiguille, **caractérisé en ce que** le panneau de base(1) est conformé de fabrication en sorte que les deux autres branches latérales opposées (1b, 1d) du panneau aient une courbure facilitant l'application de ces branches sur la peau au droit de la chambre implantée afin de maintenir la chambre lorsque l'opérateur retire l'aiguille avec son autre main, et le panneau porte-aiguille (2) et le panneau de recouvrement (3) présentent de fabrication une courbure en sens inverse de la courbure desdites branches (1b, 1d) de façon à épouser la courbure des branches lorsqu'ils sont rabattus sur le panneau de base.

2. Dispositif selon la revendication 1 qui comporte un disque (8) en matière plastique très dure rapporté et fixé sur l'une (1d) des branches latérales précourbées du panneau de base (1), ce disque présentant un relief (9) choisi pour empêcher un glissement de la pointe de l'aiguille lorsque cette pointe est amenée au contact du disque après escamotage de l'aiguille dans le dispositif.

3. Dispositif selon la revendication 1 ou 2 et dans lequel les branches courbables opposées (1c, 1e) du panneau de base (1) présentent des reliefs (5) facilitant l'application des doigts sur ces branches.

4. Dispositif selon l'une des revendications 1 à 3 dans lequel les oreilles relevables (2a, 2b) du panneau porte-aiguille (2) sont munies de moyens (11) coopérant pour maintenir à la demande les deux oreilles appliquées l'une contre l'autre.

5. Dispositif selon l'une des revendications 1 à 4 dans lequel le panneau de recouvrement (3) est conformé pour constituer une gouttière (7) apte à recevoir un adhésif et à recouvrir la branche proximale (P) de l'aiguille lorsque ce panneau est appliqué sur le panneau porte-aiguille.

6. Dispositif selon l'une des revendications 1 à 5 dans lequel ladite paroi est formée d'une feuille en matière plastique souple découpée et préformée.

7. Dispositif selon l'une des revendications 1 à 6 livré dans une pochette où la paroi est sensiblement mise à plat.

8. Dispositif selon la revendication 7 et qui comprend également à l'intérieur de la pochette l'aiguille et un capuchon pour protéger le biseau de l'aiguille.

## Claims

1. Anti-stick device for safely maneuvering an injection needle through the skin for the purpose of feeding a chamber implanted under the skin, this needle being bent and having a perforating distal branch and a proximal feed branch which forms a bend with the perforating branch, this device being composed of a wall formed by articulated panels (1, 2, 3) which allow the wall to be brought into a configuration in which one of the panels called the needle-holding panel (2) is folded down onto another panel (1) called the base panel and in which a third panel (3) called the covering panel is folded down onto the needle-holding panel and fixed to it, and to be brought into a configuration in which the needle-holding panel and the covering panel fixed to one another are distanced from the base panel and form, between themselves and said base panel, a space which is sufficient to contain the distal branch (D) of the needle, the base panel (1) and the needle-holding panel (2) having respective holes (4, 6) which permit passage of the distal branch of the needle and which coincide when the panels are applied onto one another, in such a way that the distal branch can be introduced into the holes of the panels folded down one on top of the other until the proximal branch of the needle rests on the needle-holding panel, the covering panel being able to cover the proximal branch (P) of the needle when it is folded down onto the needle-holding panel, the base panel (1) determining a central zone (1a) including said hole (4) of the panel and four lateral branches lying opposite one another in pairs and perpendicular to one another in pairs, two opposite lateral branches (1c, 1e) of the panel being capable of being bent at will under the pressure exerted by two fingers of a hand in order to press these branches onto the skin and the chamber and the needle-holding panel (2) and the covering panel (3) being contiguous, respectively, with one or other of the branches (1b,' 1d) of the base panel, and the needle-holding panel (2) forming two lateral lugs (2a, 2b) which can be lifted to permit manual gripping of the device at the time of puncture and at the time of withdrawal of the needle, **characterized in that** the base panel (1) is manufactured in such a shape that the other two opposite lateral branches (1b, 1d) of the panel have a curvature facilitating application of these branches on the skin in line with the implanted chamber so as to hold the chamber in place when the operator withdraws the needle with his other hand, and the needle-holding panel (2) and the covering panel (3) having, from manufacture, a curvature which is the opposite of the curvature of said branches (1b, 1d) so as to match the curvature of the branches when they are folded down onto the base panel.

2. Device according to Claim 1, which comprises a disk (8) of very hard plastic material attached to and fixed on one (1d) of the pre-curved lateral branches of the base panel (1), this disk having a relief (9) chosen to prevent slipping of the tip of the needle when this tip is brought into contact with the disk after retraction of the needle into the device.

3. Device according to Claim 1 or Claim 2, and in which the opposite bendable branches (1c, 1e) of the base panel (1) have reliefs (5) facilitating application of the fingers on these branches.

4. Device according to one of Claims 1 to 3, in which the liftable lugs (2a, 2b) of the needle-holding panel (2) are equipped with means (11) which cooperate in order to keep the two lugs applied against one another when so desired.

5. Device according to one of Claims 1 to 4, in which the covering panel (3) is shaped to constitute a channel (7) able to receive an adhesive and to cover the proximal branch (P) of the needle when this panel is applied to the needle-holding panel.

6. Device according to one of Claims 1 to 5, in which said wall is formed by a sheet of flexible plastic material which has been cut out and pre-formed.

7. Device according to one of Claims 1 to 6, supplied in a pouch in which the wall is laid substantially flat.

8. Device according to Claim 7 and comprising, also inside the pouch, the needle and a cap for shielding the beveled edge of the needle.

## Patentansprüche

1. Anti-Stechvorrichtung zum sicheren Handhaben einer Injektionsnadel durch die Haut zum Einspeisen einer unter der Haut implantierten Kammer, wobei diese Nadel abgewinkelt ist und einen distalen, perforierenden Schenkel sowie einen proximalen Schenkel zum Einspeisen aufweist, der zum perforierenden Arm hin eine Abwinklung bildet, diese Vorrichtung von einer Wand gebildet ist, die aus gelenkig verbundenen Platten (1, 2, 3) geformt ist, die es der Wand gestatten, in eine Anordnung gebracht zu werden, wo die eine der Platten, die Nadelträgerplatte (2) genannt wird, auf eine andere Platte (1), die Grundplatte genannt wird, umgeschlagen ist, und wo eine dritte Platte (3), die Abdeckplatte genannt wird, auf die Nadelträgerplatte umgeschlagen ist und an dieser befestigt ist, und in eine Anordnung gebracht zu werden, wo die Nadelträgerplatte und die Abdeckplatte, die aneinander befestigt sind, von der Grundplatte abgespreizt sind und zwischen ihnen und dieser einen Raum aussparen, der ausreicht, um den distalen Schenkel (D) der Nadel aufzunehmen, die Grundplatte (1) und die Nadelträgerplatte (2) jeweilige Perforierungen (4, 6) aufweisen, die den Durchtritt des distalen Schenkels der Nadel gestatten und die übereinanderliegen, wenn die Platten aufeinandergelegt sind, derart, dass der distale Schenkel in die Perforierungen der aufeinander umgeschlagenen Schenkel eingeführt werden kann, bis der proximale Schenkel der Nadel auf der Nadelträgerplatte ruht, die Abdeckplatte geeignet ist, den proximalen Schenkel (P) der Nadel abzudecken, wenn sie über die Nadelträgerplatte zurückgeschlagen ist, die Grundplatte (1) eine mittige Zone (1a), die die genannte Perforierung (4) der Platte umfasst, und vier Seitenschenkel bestimmt, die paarweise gegenüberliegen und paarweise senkrecht stehen, von denen zwei gegenüberliegende Seitenschenkel (1c, 1e) der Platte auf Wunsch unter dem Druck zweier Finger einer Hand krümmbar sein sollen, um diese Schenkel auf der Haut und der Kammer anzudrücken, und die Nadelträgerplatte (2) und die Abdeckplatte (3) mit dem Schenkel (1b, 1d) der Nadelträgerplatte (2) bzw. der Grundplatte (3), eine mit dem anderen, zusammenfügbar sein sollen, und die Nadelträgerplatte (2) zwei seitliche Ohren (2a, 2b) bildet, die aufstellbar sind, um zum Ergreifen der Vorrichtung mit der Hand während der Punktierung und während des Zurückziehens der Nadel zu dienen, **dadurch gekennzeichnet, dass** die Grundplatte (1) herstellungsmäßig derart ausgebildet ist, dass die beiden anderen seitlichen gegenüberliegenden Schenkel (1b, 1d) der Platte eine Krümmung haben, die das Andrücken dieser Schenkel auf der Haut über der implantierten Kammer erleichtert, um die Kammer zu halten, wenn die behandelnde Person die Nadel mit ihrer anderen Hand zurückzieht, und die Nadelträgerplatte (2) und die Abdeckplatte (3) von der Herstellung eine Krümmung in zur Krümmung der genannten Schenkel (1b, 1d) umgekehrter Richtung aufweisen, derart, dass sie der Krümmung der Schenkel angepasst ist, wenn diese auf die Grundplatte umgeschlagen sind.

2. Vorrichtung nach Anspruch 1, die eine Scheibe (8) aus sehr hartem Kunststoff umfasst, die auf dem einen (1d) der seitlichen vorgekrümmten Schenkel der Grundplatte (1) aufgesetzt und befestigt ist, wobei diese Scheibe ein Relief (9) aufweist, das ausgewählt ist, um ein Gleiten der Spitze der Nadel zu verhindern, wenn diese Spitze nach dem Ausklappen der Nadel in der Vorrichtung in Berührung mit der Scheibe gebracht wird.

3. Vorrichtung nach Anspruch 1 oder 2, in der die krümmbaren, gegenüberliegenden Schenkel (1c, 1e) der Grundplatte (1) Reliefs (5) aufweisen, die das Aufdrücken der Finger auf diese Schenkel erleichtern.

4. Vorrichtung nach einem der Ansprüche 1 bis 3, in der die aufstellbaren Ohren (2a, 2b) der Nadelträgerplatte (2) mit Mitteln (11) versehen sind, die zusammenwirken, um auf Wunsch die beiden Ohren gegeneinander angelegt zu halten.

5. Vorrichtung nach einem der Ansprüche 1 bis 4, worin die Abdeckplatte (3) zum Bilden einer Rinne (7) ausgebildet ist, die geeignet ist, einen Klebstoff aufzunehmen und den proximalen Schenkel (P) der Nadel abzudecken, wenn diese Platte gegen die Nadelträgerplatte angelegt wird.

6. Vorrichtung nach einem der Ansprüche 1 bis 5, worin die genannte Wand aus einem zugeschnittenen und vorgeformten Bogen aus weichem Kunststoff geformt ist.

7. Vorrichtung nach einem der Ansprüche 1 bis 6, geliefert in einem Täschchen, wo die Wand im Wesentlichen eben liegt.

8. Vorrichtung nach Anspruch 7, die im Inneren des Täschchens auch die Nadel und eine Kappe umfasst, um die Abschrägung der Nadel zu schützen.
